(19) Europäisches Patentamt
European Patent Office
Office européen des brevets

(11) Veröffentlichungsnummer: **0 092 706**
**A1**

(12) # EUROPÄISCHE PATENTANMELDUNG

(21) Anmeldenummer: 83103372.5

(22) Anmeldetag: 07.04.83

(51) Int. Cl.³: C 07 D 413/12
A 01 N 43/72

(30) Priorität: 20.04.82 JP 64700/82

(43) Veröffentlichungstag der Anmeldung: 02.11.83 Patentblatt 83/44

(84) Benannte Vertragsstaaten: AT BE CH DE FR GB IT LI NL

(71) Anmelder: NIHON TOKUSHU NOYAKU SEIZO K.K.
No.4, 2-Chome, Nihonbashi Honcho
Chuo-ku Tokyo 103(JP)

(72) Erfinder: Aya, Masahiro
2-844, Suzuki-cho
Kodaira-shi Tokyo(JP)

(72) Erfinder: Saito, Junichi
3-7-12, Osawa
Mitaka-shi Tokyo(JP)

(72) Erfinder: Kume, Toyohiko
6-7-8, Asahigaoka
Hino-shi Tokyo(JP)

(72) Erfinder: Yasui, Kazuomi
7-6-15, Shakujii-dai
Nerima-ku Tokyo(JP)

(74) Vertreter: Adrian, Albert, Dr. et al,
BAYER AG c/o Zentralbereich Patente Marken und Lizenzen
D-5090 Leverkusen 1, Bayerwerk(DE)

(54) **Oxadiazol-Derivate.**

(57) Neue Oxadiazol-Derivate der Formel

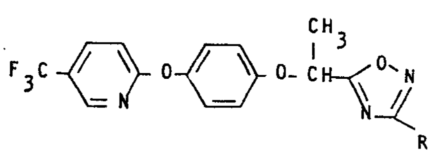

(I)

in der

R eine niedere Alkyl-Gruppe, eine Nieder-Alkoxy-Nieder-Alkyl-Gruppe, eine Aralkyl-Gruppe oder eine Aryloxyalkyl-Gruppe bezeichnet,

mehrere Verfahren zur Herstellung der neuen Stoffe und deren Verwendung zur Bekämpfung von Unkräutern.

EP 0 092 706 A1

0092706 Da/MH
I b

NIHON TOKUSHU NOYAKU SEIZO K.K. / Tokyo/Japan

## Oxadiazol-Derivate

Die vorliegende Erfindung betrifft neue Oxadiazol-Derivate, mehrere Verfahren zu ihrer Herstellung und ihre Verwendung als Herbizide.

Die vorliegende Erfindung betrifft Oxadiazol-Derivate, die durch die nachstehende allgemeine Formel (I) bezeichnet sind:

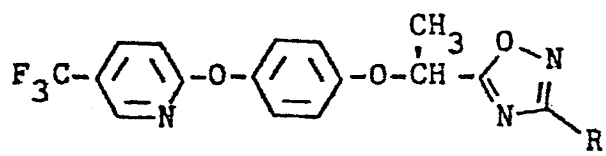

(I)

In der Formel bezeichnet R eine niedere Alkyl-Gruppe, eine Nieder-Alkoxy-Nieder-Alkyl-Gruppe, eine Aralkyl-Gruppe oder eine Aryloxyalkyl-Gruppe.

Die Oxadiazol-Derivate der allgemeinen Formel (I) können beispielsweise mittels des nachstehenden Verfahrens hergestellt werden, und die vorliegende Erfindung betrifft ebenfalls dieses Verfahren zur Herstellung von Oxadiazol-Derivaten der im Vorstehenden angegebenen

allgemeinen Formel (I). Das Verfahren ist dadurch gekennzeichnet, daß eine Verbindung der Formel

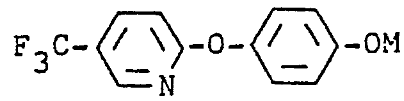

(II),

in der

M für ein Wasserstoff-Atom oder ein Alkalimetall-Atom steht,

mit einer Verbindung der allgemeinen Formel (III)

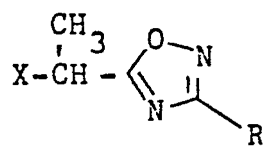

(III)

,

in der

R die im Vorstehenden angegebene Bedeutung hat und

X für ein Halogen-Atom steht,

zur Reaktion gebracht wird.

Die vorliegende Erfindung betrifft außerdem die Verwendung von Oxadiazol-Derivaten der allgemeinen Formel (I) als Herbizide.

Die JP-OS 57774/1981 (offengelegt am 20. Mai 1981) gibt an, daß 3-Methyl-5-{1-/¯4-(2-chloro-4-trifluoromethyl-phenoxy)-phenoxy_7ethyl}-1,2,4-oxadiazol der nachstehenden Formel

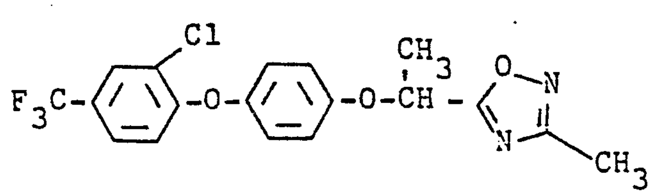

herbizide Aktivität besitzt.

Seitens der Anmelderin wurden Untersuchungen mit dem Ziel durchgeführt, neue Verbindungen aufzufinden, die eine besondere herbizide Wirksamkeit gegenüber Unkräutern der Familie der Gramineae entfalten.

Diese Untersuchungen haben zu dem Ergebnis geführt, daß durch die vorstehende Formel (I) bezeichnete Oxadiazol-Derivate, die bisher in der Literatur nicht beschrieben sind, hergestellt werden können und diese neuen Verbindungen eine hervorragende herbizide Wirksamkeit besitzen, die der vorgenannten Zielsetzung entspricht.

Die seitens der Anmelderin durchgeführten Untersuchungen haben ergeben, daß die durch die vorstehende allgemeine Formel (I) bezeichneten Verbindungen der vorliegenden Erfindung eine ausgezeichnete selektive herbizide Wirksamkeit besitzen und eine ausgezeichnete Langzeit-Hemmwirkung gegenüber der Fortpflanzung der perennierenden Unkräuter der Familie der Gramineae ausüben, die nur schwer zu bekämpfen sind. Darüber hinaus wurde gefunden, wie aus den Ergebnissen der im Folgenden angegebenen Tests hervorgeht, daß die Verbindungen gemäß der vorliegenden Erfindung einzigartige Verbindungen sind, die sicher gegen ein breites Spektrum von Acker-Unkräutern eingesetzt werden können, ohne daß sie Phytotoxizität gegenüber wertvollen breitblättrigen Nutzpflanzen aufweisen.

Die Verbindungen der Formel (I) sind hinsichtlicher ihrer Struktur dadurch charakterisiert, daß der in der allgemeinen Formel (I) bezeichnete Substituent R sich in der 3-Stellung des 1,2,4-Oxadiazol-Rings befindet und eine 1-/¯4-(5-Trifluoromethyl-2-pyridyloxy)phenoxy_7-ethyl-Gruppe in der 5-Stellung des vorerwähnten hetero-

Nit 154

cyclischen Rings substituiert ist. Es wurde gefunden, daß die Verbindungen der vorliegenden Erfindung aufgrund der speziellen, oben bezeichneten chemischen Struktur eine hervorragende herbizide Wirkung besitzen, die die analoge, in der im Vorstehenden zitierten JP-OS beschriebene Verbindung nicht aufzuweisen vermag, wie im Folgenden durch ein Vergleichsbeispiel belegt wird.

Ziel der vorliegenden Erfindung sind demgemäß neue Oxadiazol-Derivate der allgemeinen Formel (I), mehrere Verfahren zu deren Herstellung sowie deren Verwendung als Herbizide.

Diese und andere Ziele und Vorteile sind der folgenden Beschreibung zu entnehmen.

Die Herbizide gemäß der vorliegenden Erfindung können mit Vorteil zur Unkrautbekämpfung eingesetzt werden, da sie als charakteristische Eigenschaften eine niedrige Toxizität gegenüber warmblütigen Tieren und eine gute Selektivität für Kulturpflanzen besitzen (das heißt, bei der Anwendung in den üblichen Konzentrationen keine Phytotoxizität gegenüber Kulturpflanzen aufweisen). Insbesondere zeigen die Herbizide gemäß der vorliegenden Erfindung eine herausragende selektive Bekämpfungswirksamkeit, wenn sie als Mittel für die Bodenbehandlung vor dem Auflaufen und als Mittel für die Behandlung von Stengeln und Laub sowie des Bodens gegen Unkräuter aus der Familie der Gramineae verwendet werden.

Wie bereits oben dargelegt wurde, zeichnen sich die Verbindungen gemäß der vorliegenden Erfindung durch hohe Sicherheit und hervorragende herbizide Wirkungen aus.

Die Verbindungen gemäß der vorliegenden Erfindung besitzen ein solches herbizides Spektrum, daß sie eine starke herbizide Wirkung gegen

Echinochloa crus-galli P. Beauv.,
Digitaria adscendens Henr.,
Eleusine indica Gaertn.,
Setaria viridis P. Beauv.,
Avena fatua,
Alopecurus aequalis var. amurensis Ohwi,
Setaria lutescens,
Agropyron repens und
Agropyron tsukushiense var. transiens Ohwi

sowie auch eine ausgezeichnete herbizide Wirkung und fortflanzungshemmende Wirkung gegenüber perennierenden Unkräutern wie

Johnson-Gras und
Cynodon dactylon.

Die Herbizide der vorliegenden Erfindung können ohne Phytotoxizität beim Anbau vieler Nutzpflanzen wie Bohnen, Baumwolle, Möhren, Kartoffeln, Rüben, Kohlgemüsen, Senf, Erdnüssen, Rettich, Tabak, Tomaten und Gurken eingesetzt werden.

Die Verbindungen der Formel (I) gemäß der vorliegenden Erfindung können beispielsweise nach dem folgenden Verfahren i) hergestellt werden.

Verfahren i)

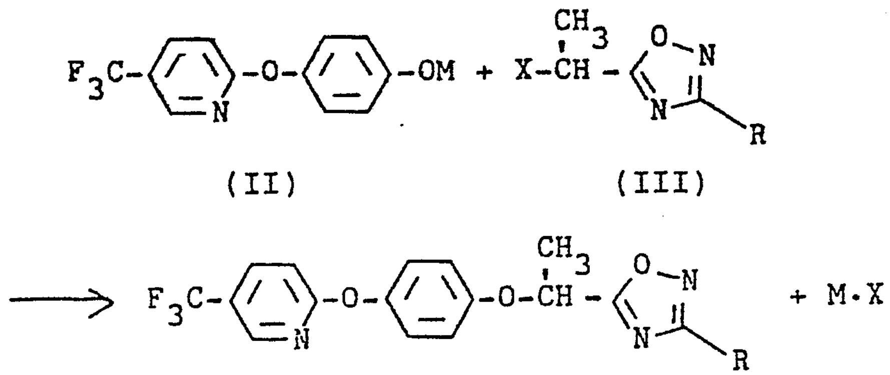

(II) (III)

(I)

In diesen Formeln haben R, X und M die im Vorstehenden angegebenen Bedeutungen.

In dem obigen Reaktionsschema bezeichnet R eine niedere Alkyl-Gruppe wie Methyl, Ethyl, Propyl, Isopropyl oder n-(iso-, sec- oder tert-)Butyl, eine Nieder-Alkoxy-Nieder-Alkyl-Gruppe mit den gleichen niederen Alkyl-Gruppen, wie sie hiervor beispielhaft angegeben sind, eine Aralkyl-Gruppe, beispielsweise eine Phenyl-Nieder-Alkyl-Gruppe wie Benzyl oder Phenetyl, oder eine Aryloxyalkyl-Gruppe, beispielsweise eine Phenoxy-Nieder-Alkyl-Gruppe wie Phenoxymethyl oder Phenoxyethyl oder α-Naphthoxymethyl.

Beispiele für X sind solche Halogen-Atome wie Fluor, Chlor, Brom und Iod, vorzugsweise Chlor und Brom.

M bezeichnet ein Wasserstoff- oder ein Alkalimetall-Atom wie Lithium, Natrium oder Kalium.

Spezielle Beispiele für die Ausgangs-Verbindung der allgemeinen Formel (II), die bei der Herstellung der

Verbindungen der vorliegenden Erfindung entsprechend dem vorstehenden Reaktionsschema eingesetzt wird, umfassen 4-(5-Trifluoromethyl-2-pyridyloxy)phenol und seine Na-, K- und Li-Salze.

In gleicher Weise umfassen spezielle Beispiele für die Ausgangs-Verbindung der allgemeinen Formel (III) in dem vorstehenden Reaktionsschema

5(1-Bromoethyl)-3-methyl-1,2,4-oxadiazol,
5(1-Bromoethyl)-3-ethyl-1,2,4-oxadiazol,
5(1-Bromoethyl)-3-propyl-1,2,4-oxadiazol,
5(1-Bromoethyl)-3-isopropyl-1,2,4-oxadiazol,
5(1-Bromoethyl)-3-tert-butyl-1,2,4-oxadiazol,
5(1-Bromoethyl)-3-methoxymethyl-1,2,4-oxadiazol,
5(1-Bromoethyl)-3-phenoxymethyl-1,2,4-oxadiazol,
5(1-Bromoethyl)-3-benzyl-1,2,4-oxadiazol und,
5(1-Bromoethyl)-3-phenetyl-1,2,4-oxadiazol.

Die entsprechenden 5(1-Chloroethyl)-Verbindungen können ebenfalls eingesetzt werden.

Das vorerwähnte Verfahren i) wird anhand eines typischen Beispiels erläutert.

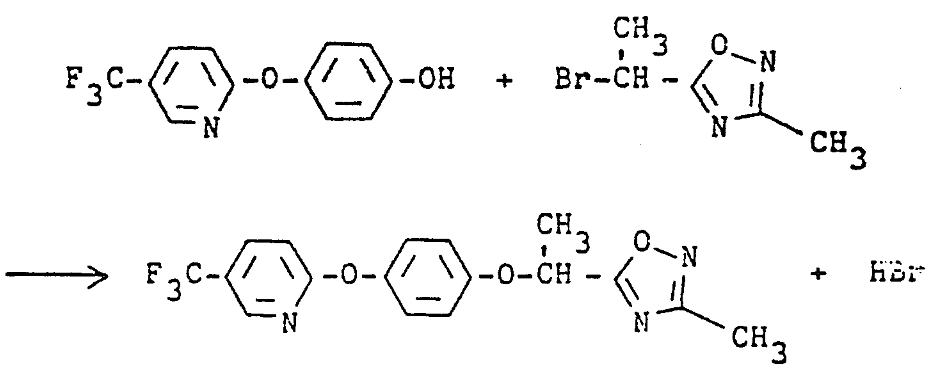

Das Verfahren zur Herstellung der Verbindung der vorliegenden Erfindung kann zweckmäßigerweise unter Verwendung eines Lösungsmittels oder Verdünnunsgmittels durchgeführt werden, und zu diesem Zweck können alle inerten Lösungsmittel oder Verdünnungsmittel verwendet werden.

Beispiele für solche Lösungsmittel oder Verdünnungsmittel sind Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die Reaktion in der vorliegenden Erfindung kann in Gegenwart eines säurebindenden Mittels durchgeführt werden. Beispiele für solche säurebindenden Mittel sind die Hydroxide, Carbonate, Hydrogencarbonate und Alkoholate von Alkalimetallen sowie tertiäre Amine wie Triethylamin, Diethylanilin und Pyridin.

Das Verfahren gemäß der vorliegenden Erfindung kann bei einer Temperatur innerhalb eines weiten Bereichs durchgeführt werden, im allgemeinen bei einer Temperatur zwischen etwa -20°C und dem Siedepunkt der Mischung,

vorzugsweise zwischen etwa 0°C und etwa 100°C. Zweckmäßigerweise wird die Reaktion unter atmosphärischem Druck durchgeführt, jedoch ist es auch möglich, unter erhöhtem oder vermindertem Druck zu arbeiten.

Die erfindungsgemäßen Verbindungen können auch nach dem folgenden Verfahren ii) und iii) hergestellt werden:

<u>Verfahren ii)</u>

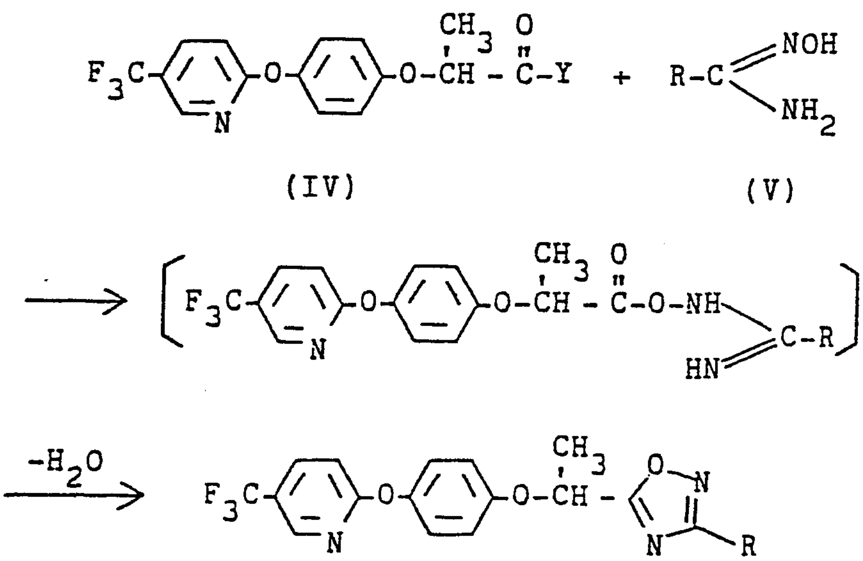


(IV) (V)

(I)

In den Formeln hat R die im Vorstehenden angegebene Bedeutung, und Y steht für eine Hydroxyl-Gruppe oder ein Halogen-Atom.

In dem vorstehenden Reaktionsschema sind spezielle Beispiele für die Ausgangs-Verbindung der allgemeinen Formel (IV) 2-/¯4-(5-Trifluoromethyl-2-pyridyloxy)phenoxy_7propionylchlorid, das ihm entsprechende Bromid und die ihm entsprechende freie Propionsäure.

Spezielle Beispiele für die Ausgangs-Verbindung der allgemeinen Formel (V) umfassen Acetamidoxim, Propionamidoxim, Butyramidoxim, Isobutyramidoxim, Pivalamidoxim, Methoxyacetamidoxim, Phenoxyacetamidoxim und α-Phenylacetamidoxim.

Verfahren iii)

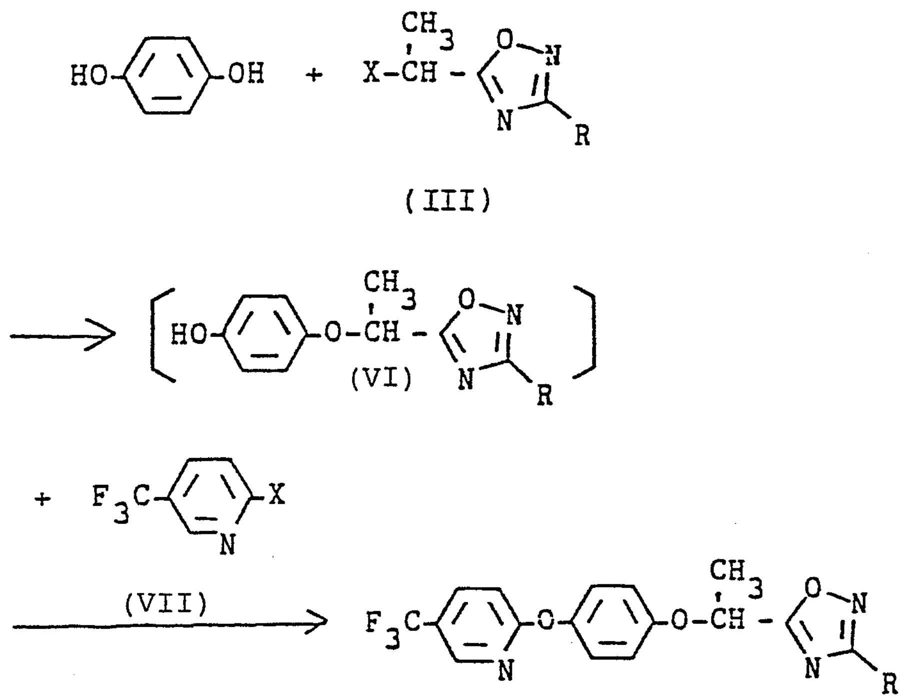


(III)

(VI)

(I)

In den Formeln haben R und X die im Vorstehenden angegebenen Bedeutungen.

In dem obigen Reaktionsschema sind spezielle Beispiele für die Ausgangs-Verbindung der allgemeinen Formel (III) diejenigen, die im Vorstehenden aufgeführt sind. Spezielle Beispiele für die Ausgangs-Verbindung der Formel (VI) umfassen 2-Chloro(oder Fluoro oder Bromo)-5-trifluoromethylpyridin.

Nit 154

Die Verfahren ii) und iii) werden nunmehr anhand spezieller Beispiele erläutert.

ii)

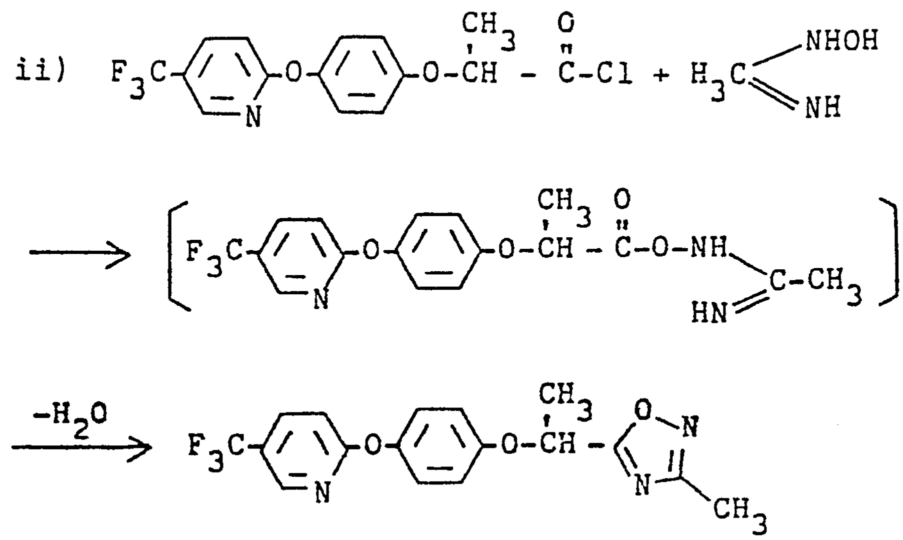


iii)

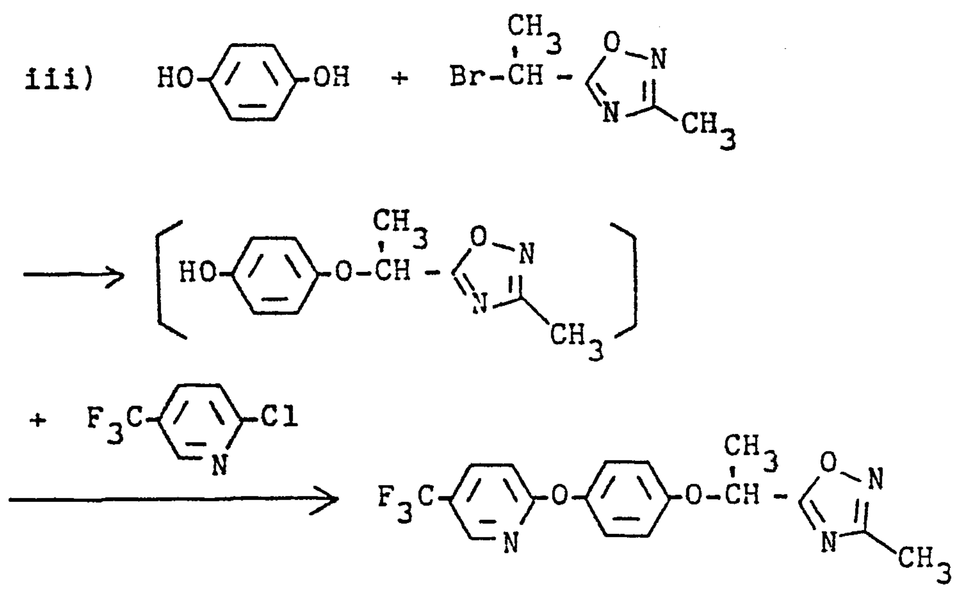

Nach Durchführung der Verfahren ii) und iii) unter Verwendung der gleichen inerten Lösungsmittel oder Verdünnungsmittel, wie sie im Vorstehenden aufgeführt sind, lassen sich die angestrebten Produkte in hoher Reinheit und in hohen Ausbeuten erhalten. Jedes der Verfahren ii) und iii) kann kontinuierlich ohne Isolierung der Zwischenprodukte durchgeführt werden.

Bei den Verfahren ii) und iii) zur Herstellung der erfindungsgemäßen Stoffe kann eine Temperatur innerhalb eines weiten Bereichs angewandt werden. Im allgemeinen wird die Reaktion bei einer Temperatur zwischen etwa -20°C und dem Siedepunkt der Mischung, vorzugsweise zwischen etwa 0°C und etwa 100°C durchgeführt. Zweckmäßigerweise wird die Reaktion unter atmosphärischem Druck durchgeführt, jedoch ist es auch möglich, unter erhöhtem oder vermindertem Druck zu arbeiten.

Für den Einsatz als Herbizid können die Verbindungen gemäß der vorliegenden Erfindung zu verschiedenen Wirkstoffpräparaten formuliert werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden die herbiziden Mittel in Form ihrer verschiedenen Präparate entweder unmittelbar oder nach dem Verdünnen mit Wasser auf die gewünschte Konzentration eingesetzt.

Beispiele für die landwirtschaftlich unbedenklichen Hilfsstoffe, auf die hier Bezug genommen wird, sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /¯z.B. n-Hexan, Petrolether, Naphtha, Erdöl-Fraktionen

(z.B. Paraffinwachse, Kerosin, Leichtöle, Mittelöle, Schweröle), Benzol, Toluol und Xylole_7, halogenierte Kohlenwasserstoffe (z.B. Methylenchlorid, Tetrachlorkohlenstoff, Trichlorethylen, Ethylenchlorid, Ethylendichlorid, Chlorbenzol und Chloroform), Alkohole (z.B. Methylalkohol, Ethylalkohol, Propylalkohol und Ethylenglycol), Ether (z.B. Ethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylschwefelsäureester (z.B. Natriumlaurylsulfat), Arylsulfonsäure-Salze (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), wirkungsverlängernde Mittel, Dispersions-Stabilisatoren (z.B. Casein, Tragant, Carboxymethylcellulose und Polyvinylalkohol) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öle, benetzbare Pulver, lösliche Pulver, Suspensionen, Stäubemittel, Granulate und pulvrige Mittel.

Die Herbizide gemäß der vorliegenden Erfindung können von etwa 0,001 bis etwa 100 Gew.-%, vorzugsweise etwa von 0,005 bis 95 Gew.-% des vorerwähnten Wirkstoffes enthalten.

Für den praktischen Gebrauch beträgt die geeignete Menge der aktiven Verbindung in den vorgenannten Mitteln

in ihren verschiedenen Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens der Unkräuter etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, Fungiziden, Mitiziden, Nematiziden, Anti-Virus-Mitteln, anderen Herbiziden, Pflanzen-Wachstumsregulatoren und Lockstoffen /¯Organophosphorester-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die erfindungsgemäße Wirkstoffe enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Dispergieren (Versprühen von Flüssigkeiten, Vernebeln, Zerstäuben, Stäuben, Streuen von Granulat, Wasser-Oberflächenbehandlung und Gießen) und Bodenbehandlung (Vermischen mit dem Boden und Streuen)..

Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,01 bis etwa 5,0 kg/ha, vorzugsweise etwa 0,05 bis etwa 2,0 kg/ha. In besonders gelagerten Fällen können oder sollten sogar die Aufwandmengen außerhalb des angegebenen Bereichs liegen.

Gemäß der vorliegenden Erfindung kann ein herbizides Mittel zur Verfügung gestellt werden, das als Wirkstoff eine Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung betrifft weiterhin ein Verfahren zur Unkrautbekämpfung, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Unkräuter und/oder oder ihren Lebensraum aufgebracht wird.

Die vorliegende Erfindung wird durch die folgenden Beispiele näher erläutert, ist jedoch nicht auf diese beschränkt.

Beispiel 1

Benetzbares Pulver.

15 Teile der erfindungsgemäßen Verbindung Nr. 1, 80 Teile eines Gemisches (1:5) aus Weißruß (einem feinen Pulver aus wasserhaltigem, amorphen Siliciumdioxid) und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/oder ihren Lebensraum aufgebracht.

Beispiel 2

Emulgierbares Konzentrat.

30 Teile der erfindungsgemäßen Verbindung Nr. 2, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgebracht.

Beispiel 3

Stäubemittel.

2 Teile der erfindungsgemäßen Verbindung Nr. 3 und 98 Teile Tonpulver wurden pulverisiert und gemischt, wodurch ein Stäubemittel hergestellt wird. Dieses wird auf Unkräuter und/oder ihren Lebensraum aufgebracht.

Beispiel 4

Stäubemittel.

Die erfindungsgemäße Verbindung Nr. 4 (1,5 Teile), 0,5 Teile Isopropylhydrogenphosphat und 98 Teile Tonpulver wurden pulverisiert und gemischt, wodurch ein Stäubemittel hergestellt wurde. Dieses wird auf Unkräuter und/ oder ihren Lebensraum aufgebracht.

Beispiel 5

Granulat.

Wasser (25 Teile) wird zu einer Mischung aus 10 Teilen der erfindungsgemäßen Verbindung Nr. 1, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wird. Dieses wird auf Unkräuter und/oder ihren Lebensraum aufgebracht.

Beispiel 6

Granulat.

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Gew.-Teile der öligen erfindungsgemäßen Verbindung Nr. 2 zur gleichmäßigen Benetzung auf die Tonmineral-Teilchen aufgesprüht. Das erhaltene Granulat wird auf Unkräuter und/oder ihren Lebensraum aufgebracht.

Nit 154

0092706

Die ausgezeichnete Wirkung der Verbindungen gemäß der vorliegenden Erfindung ist aus den Ergebnissen der im Folgenden beschriebenen Tests zu entnehmen.

Test-Beispiel 1

Test der Vorauflauf-Bodenbehandlung an Acker-Unkräutern und -Nutzpflanzen:

(1) Herstellung der Zusammensetzungen der aktiven Verbindungen:

Träger: 5 Gew.-Teile Aceton;
Emulgator: 1 Gew.-Teil Benzyloxypolyglycolether.

Jede der Zusammensetzungen wurde durch Vermischen von 1 Gew.-Teil jeweils einer der in der Tabelle 1 aufgeführten wirksamen Verbindungen mit dem Träger und dem Emulgator hergestellt, wodurch ein emulgierbares Konzentrat erhalten wurde. Eine vorher festgelegte Menge des emulgierbaren Konzentrats wurde mit Wasser verdünnt, wodurch ein Test-Präparat erhalten wurde.

(2) Test-Verfahren:

In einem Gewächshaus wurden Samen von Erdnüssen, Garten-Erbsen, Sojabohnen und Baumwolle in Töpfe (1000 $cm^2$) eingesät, die mit Ackerboden gefüllt waren. Boden, der Samen von Agropyron repens, Echinochloa crus-galli P. Beauv. und Setaria lutescens enthielt, wurde in einer Tiefe von 1 cm darüber gegeben.

Einen Tag nach der Einsaat und dem Bedecken mit dem Boden wurden 10 ml jedes Test-Präparats mit einer Konzentration desselben von 100 ppm gleichmäßig auf die Oberflächenschicht des Bodens in jedem der Test-Töpfe aufgesprüht.

Vier Wochen nach Aufsprühen des Präparats wurden die herbizide Wirkung und die Phytotoxizität der Test-Präparate untersucht.

Bewertung der herbiziden Wirkung (Unkrautvernichtungsrate bezogen auf die unbehandelten Flächen):

| | |
|---|---|
| 10 | 100 % (vollständig verdorrt) |
| 9 | mindestens 90 %, jedoch weniger als 100 % |
| 8 | mindestens 80 %, jedoch weniger als 90 % |
| 7 | mindestens 70 %, jedoch weniger als 80 % |
| 6 | mindestens 60 %, jedoch weniger als 70 % |
| 5 | mindestens 50 %, jedoch weniger als 60 % |
| 4 | mindestens 40 %, jedoch weniger als 50 % |
| 3 | mindestens 30 %, jedoch weniger als 40 % |
| 2 | mindestens 20 %, jedoch weniger als 30 % |
| 1 | mindestens 10 %, jedoch weniger als 20 % |
| 0 | weniger als 10 % (keine herbizide Wirkung) |

Bewertung der Phytotoxizität (Phytotoxizitätsrate bezogen auf die unbehandelten Flächen):

| | |
|---|---|
| 10 | mindestens 90 % (Ausrottung) |
| 9 | mindestens 80 %, jedoch weniger als 90 % |
| 8 | mindestens 70 %, jedoch weniger als 80 % |
| 7 | mindestens 60 %, jedoch weniger als 70 % |
| 6 | mindestens 50 %, jedoch weniger als 60 % |
| 5 | mindestens 40 %, jedoch weniger als 50 % |
| 4 | mindestens 30 %, jedoch weniger als 40 % |
| 3 | mindestens 20 %, jedoch weniger als 30 % |
| 2 | mindestens 10 %, jedoch weniger als 20 % |
| 1 | mehr als 0 %, jedoch weniger als 10 % |
| 0 | 0 % (keine Phytotoxizität) |

Die Testergebnisse sind in der Tabelle 1 aufgeführt.

0092706

Tabelle 1

| Verbindung Nr. | Menge des Wirkstoffs (kg/ha) | Herbizide Wirkung | | | Phytotoxizität | | | |
|---|---|---|---|---|---|---|---|---|
| | | Unkräuter | | | Nutzpflanzen | | | |
| | | Agropyron repens | Echinochloa crus-galli | Setaria lutescens | Erdnuß | Gartenerbse | Baumwolle | Sojabohne |
| 1 | 0,1 | 10 | 10 | 10 | 0 | 0 | 0 | 0 |
| 4 | 0,1 | 10 | 10 | 10 | 0 | 0 | 0 | 0 |
| 7 | 0,1 | 10 | 10 | 10 | 0 | 0 | 0 | 0 |
| Vergleich: | | | | | | | | |
| A | 0,1 | 3 | 4 | 4 | 0 | 0 | 0 | 0 |

Anmerkungen zu Tabelle 1:

1. Die Nummern der Verbindungen entsprechen denjenigen, die in dem Synthese-Beispiel 1 und in der Tabelle 5 angegeben sind.
2. Die zum Vergleich herangezogene Verbindung A ist 3-Methyl-5-{1-/¯4-(2-chloro-4-trifluoromethylphenoxy)-phenoxy_7ethyl}-1,2,4-oxadiazol der nachstehenden Formel

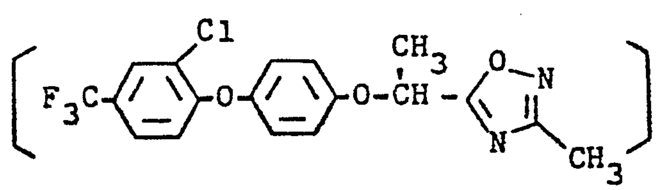

.

(die Verbindung, die in der JP-OS 57774/1981 beschrieben ist).

Nit 154

Test-Beispiel 2

Test der Stengel- und Laub-Behandlung an Acker-Unkräutern und -Nutzpflanzen:

In einem Gewächshaus wurden Samen von Sojabohnen, Rettichen und Rüben in Töpfe (2000 $cm^2$) eingesät, die mit Ackerboden gefüllt waren. Boden der Samen von Panicum crus-galli, Eleusine Indica Gaertn., Setaria viridis P. Beauv., Avena fatua und Alopecurus aequalis var. amurensis Ohwi enthielt, wurde in einer Tiefe von 1 cm darüber gegeben.

Zehn Tage nach der Einsaat und dem Bedecken mit dem Boden (als die Unkräuter sich im Durchschnitt in ihrem Zweiblatt-Stadium und Rettich-, Sojabohnen- und Rübenpflanzen sich im frühen Stadium der Entwicklung ihrer Hauptblätter befanden) wurden 20 ml jedes Test-Präparats mit einer Konzentration desselben von 200 ppm, das in gleicher Weise wie in Test-Beispiel 1 hergestellt worden war, gleichmäßig auf die Stengel und Blätter der Pflanzen gesprüht.

Drei Wochen nach dem Sprühen wurden die herbizide Wirkung und die Phytotoxizität der Test-Verbindungen in der gleichen Weise wie in dem Test-Beispiel 1 untersucht.

Die Ergebnisse sind in der Tabelle 2 aufgeführt.

Tabelle 2

| Verbindung Nr. | Menge des Wirkstoffs (kg/ha) | Herbizide Wirkung Unkräuter Panicum crus-galli | Eleusine indica | Setaria viridis | Avena fatua | Alopecurus aequalis | Phytotoxizität Nutzpflanzen Sojabohne | Rettich | Rübe |
|---|---|---|---|---|---|---|---|---|---|
| 1 | 0,2 | 10 | 10 | 10 | 10 | 10 | 0 | 0 | 0 |
| 3 | 0,2 | 10 | 10 | 10 | 10 | 10 | 0 | 0 | 0 |
| 5 | 0,2 | 10 | 10 | 10 | 10 | 10 | 0 | 0 | 0 |
| 6 | 0,2 | 10 | 10 | 10 | 10 | 10 | 0 | 0 | 0 |
| 8 | 0,2 | 10 | 10 | 10 | 10 | 10 | 0 | 0 | 0 |
| Vergleich: | | | | | | | | | |
| A | 0,2 | 5 | 3 | 4 | 3 | 4 | 0 | 0 | 0 |

Anmerkungen zu Tabelle 2:

1. Die Nummern der Verbindungen entsprechen denjenigen, die in dem Synthese-Beispiel 1 und in der Tabelle 5 angegeben sind.
2. Die zum Vergleich herangezogene Verbindung A ist die gleiche wie in Tabelle 1.

Nit 154

Test-Beispiel 3

Test der herbiziden Wirkung und der Hemmwirkung auf die Fortpflanzung gegen Agropyron tsukushiense var. transiens Ohwi:

Ein Weg zwischen Reisfeldern, auf dem Agropyron tsukushiense in Büscheln wuchs, wurde in Teilflächen von 1 $m^2$ unterteilt. Auf jeder Test-Fläche wurden 100 ml jedes Test-Präparats mit einer Konzentration von 200 ppm auf die Stengel und Blätter von Agropyron tsukushiense aufgesprüht. Zwanzig Tage nach dem Sprühen wurde die herbizide Wirkung des Test-Präparates in der gleichen Weise wie in Test-Beispiel 1 geprüft. Weiterhin wurde 40 und 60 Tage nach dem Sprühen die Hemmwirkung auf die Fortpflanzung von Agropyron tsukushiense untersucht.

Bewertung der Hemmwirkung auf die Fortpflanzung (Rate der Hemmwirkung auf die Fortpflanzung, bezogen auf die unbehandelten Flächen):

| | |
|---|---|
| 10 | 100 % (vollständige Hemmung) |
| 9 | mindestens 90 %, jedoch weniger als 100 % |
| 8 | mindestens 80 %, jedoch weniger als 90 % |
| 7 | mindestens 70 %, jedoch weniger als 80 % |
| 6 | mindestens 60 %, jedoch weniger als 70 % |
| 5 | mindestens 50 %, jedoch weniger als 60 % |
| 4 | mindestens 40 %, jedoch weniger als 50 % |
| 3 | mindestens 30 %, jedoch weniger als 40 % |
| 2 | mindestens 20 %, jedoch weniger als 30 % |
| 1 | mindestens 10 %, jedoch weniger als 20 % |
| 0 | weniger als 10 % (keine Hemmwirkung) |

Die Ergebnisse sind in der Tabelle 3 aufgeführt.

Tabelle 3

| Verbindung Nr. | Menge des Wirkstoffs (kg/ha) | Herbizide Wirkung 20 Tage nach dem Sprühen | Hemmwirkung auf die Fortpflanzung 40 Tage nach dem Sprühen | Hemmwirkung auf die Fortpflanzung 60 Tage nach dem Sprühen |
|---|---|---|---|---|
| 1 | 0,2 | 10 | 10 | 10 |
| 2 | 0,2 | 10 | 9 | 9 |
| 7 | 0,2 | 10 | 10 | 9 |
| Vergleich: | | | | |
| A | 0,2 | 4 | 1 | 0 |

Anmerkungen zu Tabelle 3:

1. Die Nummern der Verbindungen entsprechen denjenigen, die in dem Synthese-Beispiel 1 und in der Tabelle 5 angegeben sind.
2. Die zum Vergleich herangezogene Verbindung A ist die gleiche wie in Tabelle 1.

## Test-Beispiel 4

Test der herbiziden Wirkung und der Hemmwirkung auf die Fortpflanzung gegen Cynodon dactylon Persoon:

Eine landwirtschaftlich genutzte Ackerfläche, auf der Cynodon dactylon in Büscheln wuchs, wurde in Teilflächen von 1 m² unterteilt. Das Unkraut wurde mit jedem Test-Präparat in der gleichen Dosierung wie in Test-Beispiel 3 behandelt.

Zwanzig Tage nach dem Sprühen wurde die herbizide Wirkung geprüft. Weiterhin wurde 40 und 60 Tage nach dem Sprühen die Hemmwirkung untersucht. Die Auswertung der Ergebnisse dieses Tests erfolgte in der gleichen Weise wie im Vorstehenden beschrieben.

Die Ergebnisse sind in der Tabelle 4 aufgeführt.

Tabelle 4

| Verbindung Nr. | Menge des Wirkstoffs (kg/ha) | Herbizide Wirkung 20 Tage | Hemmwirkung auf die Fortpflanzung 40 Tage | 60 Tage |
|---|---|---|---|---|
| | | nach | dem Sprühen | |
| 1 | 0,2 | 10 | 10 | 10 |
| 7 | 0,2 | 10 | 10 | 9 |
| Vergleich: | | | | |
| A | 0,2 | 4 | 2 | 0 |

Anmerkungen zu Tabelle 4:

Die Nummern der Verbindungen und die zum Vergleich herangezogene Verbindung A entsprechen den Angaben in der Anmerkung zu Tabelle 3.

Die folgenden Synthese-Beispiele erläutern die Herstellung der Verbindungen gemäß der vorliegenden Erfindung.

Synthese-Beispiel 1

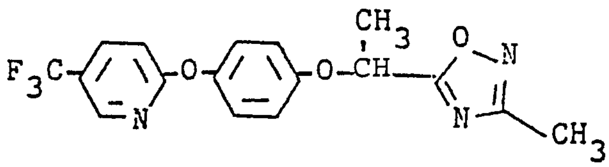

(Verbindung Nr. 1)

Unter Rühren wurden 5,2 g 5-(1-Bromoethyl)-3-methyl-1,2,4-oxadiazol tropfenweise bei einer Temperatur von 25°C bis 35°C zu einer Mischung von 7 g 4-(5-Triflucromethyl-2-pyridyloxy)phenol, 20 ml Dimethylformamid und 4 g wasserfreiem Kaliumcarbonat hinzugefügt. Die Lösung wurde 5 h bei 80°C bis 85°C gerührt und dann abgekühlt. Wasser (150 ml) wurde zugesetzt, und das entstandene ölige Produkt wurde mit 150 ml Toluol extrahiert. Der Extrakt wurde nacheinander mit einer 2-proz. wäßrigen Natriumhydroxid-Lösung und mit Wasser gewaschen und dann über wasserfreiem Natriumsulfat getrocknet. Anschließend wurde das Toluol abgedampft, wonach 9,26 g 3-Methyl-5- 1-/¯4-(5-trifluoromethyl-2-pyridyloxy)-phenoxy_7ethyl -1,2,4-oxadiazol erhalten wurden; $n_D^{20}$ 1,5200.

In analoger Weise wie in dem vorstehenden Synthese-Beispiel wurden die in der Tabelle 5 aufgeführten Verbindungen gemäß der vorliegenden Erfindung hergestellt.

Tabelle 5

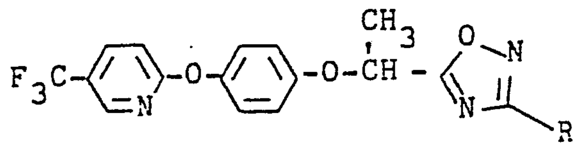

| Verbindung Nr. | R | Physikal. Konstante |
|---|---|---|
| 2 | $-C_2H_5$ | $n_D^{20}$ 1.5188 |
| 3 | $-C_3H_7$-iso | $n_D^{20}$ 1.5146 |
| 4 | $-C_3H_7$-n | $n_D^{20}$ 1.5151 |
| 5 | $-C_4H_9$-tert | Schmp. 85 - 92°C |
| 6 | $-CH_2-$ 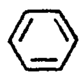 | $n_D^{20}$ 1.5472 |
| 7 | $-CH_2OCH_3$ | $n_D^{20}$ 1.5172 |
| 8 | $-CH_2-O-$ 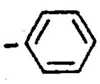 | $n_D^{20}$ 1.5518 |

Patentansprüche:

1. Oxadiazol-Derivate der allgmeinen Formel

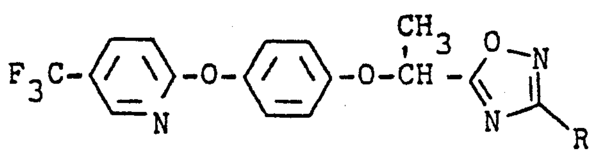 (I),

in der

R eine niedere Alkyl-Gruppe, eine Nieder-Alkoxy-Nieder-Alkyl-Gruppe, eine Aralkyl-Gruppe oder eine Aryloxyalkyl-Gruppe bezeichnet.

2. 3-Methyl-5-{1-/¯4-(5-trifluoromethyl-2-pyridyloxy)-phenoxy_7ethyl}-1,2,4-oxadiazol der Formel

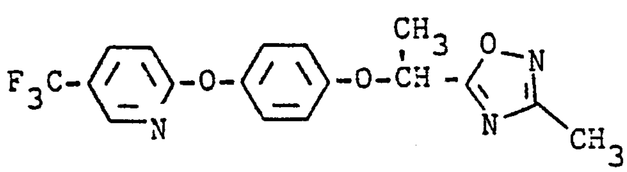

nach Anspruch 1.

Nit 154

3. 3-Ethyl-5- 1-[4-(5-trifluoromethyl-2-pyridyloxy)-phenoxy]ethyl -1,2,4-oxadiazol der Formel

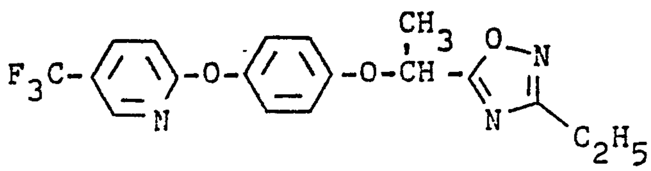

nach Anspruch 1.

4. 3-Methoxymethyl-5- 1-[4-(5-trifluoromethyl-2-pyridyl-oxy)phenoxy]ethyl -1,2,4-oxadiazol der Formel

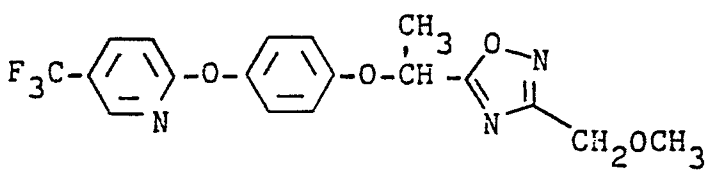

nach Anspruch 1.

5. Verfahren zur Herstellung von Oxadiazol-Derivaten der allgemeinen Formel

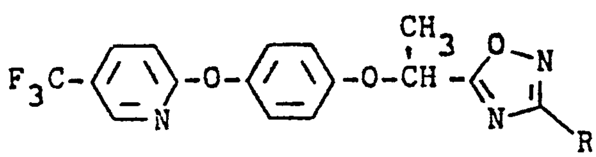 (I),

in der

R eine niedere Alkyl-Gruppe, eine Nieder-Alkoxy-Nieder-Alkyl-Gruppe, eine Aralkyl-Gruppe oder eine Aryloxyalkyl-Gruppe bezeichnet,

dadurch gekennzeichnet, daß man

a) Verbindungen der Formel

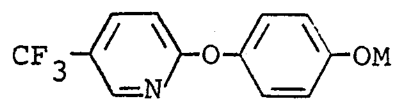 (II),

in welcher
M für ein Wasserstoff-Atom oder ein Alkalimetall-Atom steht,

mit Verbindungen der Formel

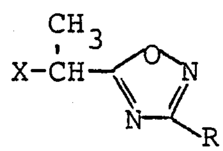 (III),

in welcher
R die oben angegebene Bedeutung hat und
X für ein Halogen-Atom steht,

umsetzt,

oder

b) Verbindungen der Formel

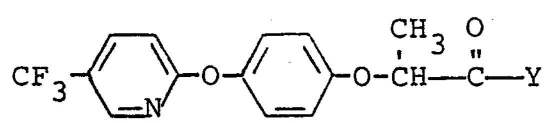 (IV),

in welcher
Y für eine Hydroxyl-Gruppe oder für ein Halogen-Atom steht,

mit Verbindungen der Formel

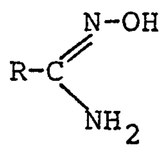 (V),

in welcher
R die oben angegebene Bedeutung hat,

umsetzt,

oder

c) Hydrochinon der Formel

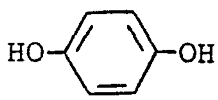

mit Verbindungen der Formel

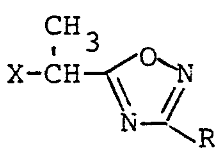 (III),

in welcher
R und X die oben angegebene Bedeutung haben,

umsetzt und die dabei entstehenden Verbindungen der Formel

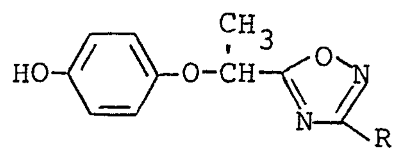

(VI),

in welcher
R die oben angegebene Bedeutung hat,

mit Verbindungen der Formel

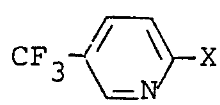

(VII),

in welcher
X die oben angegebene Bedeutung hat,

umsetzt.

6) Herbizide Mittel, gekennzeichnet durch einen Gehalt an mindestens einem Oxadiazol-Derivat der Formel (I).

7) Verwendung von Oxadiazol-Derivaten der Formel (I) zur Bekämpfung von Unkräutern.

8) Verfahren zur Bekämpfung von Unkräutern, dadurch gekennzeichnet, daß man Oxadiazol-Derivate der Formel (I) auf Unkräuter und/oder deren Lebensraum ausbringt.

9) Verfahren zur Herstellung von herbiziden Mitteln, dadurch gekennzeichnet, daß man Oxadiazol-Derivate der Formel (I) mit Streckmitteln und/oder oberflächenaktiven Stoffen vermischt.

0092706

Europäisches Patentamt

# EUROPÄISCHER RECHERCHENBERICHT

Nummer der Anmeldung

EP 83 10 3372

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,Y | CHEMICAL ABSTRACTS, Band 95, 1981, Seite 685, Nr. 203962z, Columbus, Ohio, USA & JP - A - 81 57774 (MITSUI TOATSU CHEMICALS, INC.) 20-05-1981 * Zusammenfassung * --- | 1,6 | C 07 D 413/12<br>A 01 N 43/72 |
| Y | WO-A-8 200 401 (THE DOW CHEMICAL COMPANY) * Ansprüche * --- | 1,6 | |
| Y | EP-A-0 006 608 (CIBA-GEIGY AG) * Ansprüche * --- | 1,6 | |
| A | DE-A-2 546 251 (ISHIHARA SANGYO KAISHA LTD.) * Ansprüche * ----- | 1,6 | |
| | | | RECHERCHIERTE SACHGEBIETE (Int. Cl. ³) |
| | | | C 07 D 413/00<br>C 07 D 213/00 |

Der vorliegende Recherchenbericht wurde für alle Patentansprüche erstellt.

| Recherchenort | Abschlußdatum der Recherche | Prüfer |
|---|---|---|
| DEN HAAG | 26-07-1983 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument